# FASCICULE DE BREVET EUROPEEN

(11) **EP 0 435 785 B1**
(45) Date de publication et mention de la délivrance du brevet: **08.06.1994**
(21) Numéro de dépôt: 90420557.2
(22) Date de dépôt: 20.12.1990
(51) Int. Cl.: A61K 9/50, C08J 3/12, B01J 13/02

(54) **Microsphères composites magnétisables à base d'un polymère organosilicié réticulé, leur procédé de préparation et leur application en biologie**
Magnetisierbare Komposit-Mikrokugeln bestehend aus einem vernetzten Organosilicium-Polymer, Verfahren zu ihrer Herstellung und ihre Verwendung in der Biologie
Magnetisable composite microspheres based on a crosslinked organosilicon polymer, their preparation process and their biological use

(30) Priorité: 27.12.1989 FR 8917233
(43) Date de publication de la demande: 03.07.1991
(73) Titulaire: SOCIETE PROLABO, 75011 Paris (FR)
(72) Inventeur: Charmot, Dominique, F-75019 Paris (FR); Thibon, André, F-91600 Savigny Sur Orge (FR)
(74) Mandataire: Ahner, Francis

(56) Documents cités:
- EP-A- 0 125 995
- EP-A- 0 319 828
- DE-A- 3 002 477
- FR-A- 2 618 084
- FR-A- 2 624 873

## Description

La présente invention a pour objet des microsphères composites magnétisables à base d'un polymère organosilicié réticulé, se présentant telles quelles ou en dispersion aqueuse, leur procédé de préparation et leur application en biologie.

La demanderesse a décrit dans sa demande de brevet française publiée sous le n° 2.624.873, des particules composites magnétisables à base d'organopolysiloxane réticulé, les dites particules étant constituées d'une matrice dérivant de l'hydrosilylation d'un organopolysiloxane SiVi et d'un organohydrogrenopolysiloxane SiH et encapsulées dans ladite matrice de charges magnétisables revêtues d'un agent dispersant non hydrosoluble.

Dans ce type de produits les charges magnétisables sont revêtues d'un agent dispersant rendu non hydrosoluble. La présence de cet agent dispersant peut être un inconvénient en biologie car ledit agent peut migrer vers la surface des particules et entraîner des réactions secondaires.

La demanderesse a maintenant trouvé des microsphères composites dont le coeur est constitué de charges magnétisables non recouvertes d'un agent surfactant hydrophobe réparties uniformément à l'intérieur d'un réseau de polysilsesquioxane.

Selon l'invention, il s'agit de microsphères composites magnétisables à base d'un polymère organosilicié réticulé se présentant telles quelles ou en dispersion aqueuse, caractérisées en ce qu'elles sont formées :
- d'un coeur constitué de charges magnétisables présentant une taille généralement inférieure à 300x10⁻⁴ »m, de préférence de l'ordre de 50 à 120x10⁻⁴ »m, réparties uniformément à l'intérieur d'un réseau de polysilsesquioxane (appelé SiVi) contenant par molécule au moins deux groupes éthyléniquement insaturés ("groupe SiVi") liés chacun à un atome de silicium ou à un atome de carbone d'un groupement organique relié à la chaîne polysilsesquioxane par une liaison Si-C, portant éventuellement des motifs ionogènes et/ou réactifs non éthyléniques choisis parmi les motifs amino, epoxy, mercapto et halogeno liés à un atome de silicium ou à un atome de carbone d'un groupement hydrocarboné relié à la chaîne polysilsesquioxane par une liaison Si-C ;
- et d'une écorce à base d'un polymère organosilicié réticulé dérivé de l'hydrosilylation
   . d'un organohydrogénopolysiloxane (appelé SiH) contenant par molécule au moins trois atomes d'hydrogène liés chacun à un atome de silicium ("groupes SiH"), présentant une viscosité de l'ordre de 5 à 1500 mPas à 25°C, de préférence entre 20 et 150 mPas à 25°C et portant éventuellement des motifs ionogènes et/ou réactifs non vinyliques choisis parmi les motifs amino, epoxy, mercapto et halogeno liés à un atome de silicium ou à un atome de carbone d'un groupement hydrocarboné relié à la chaîne organohydrogénopolysiloxane par une liaison Si-C
   . avec les groupes éthyléniquement insaturés dudit polysilsesquioxane SiVi.

Parmi les matériaux pouvant constituer les charges magnétisables, on peut citer la magnétite, l'hématite, le dioxyde de chrome, les ferrites telles que les ferrites de manganèse, de nickel, de manganèse-zinc.

Les matériaux préférentiels sont la magnétite et l'hématite. Ces matériaux peuvent également être présents en mélange avec des charges présentant un spectre de fluorescence, telles que oxyde ou oxysulfure d'yttrium activé à l'europium, borate de gadolinium-cérium-terbium, aluminate de cérium-terbium, aluminate de magnésium-baryum dopé à l'europium divalent.

La quantité de charges magnétisables correspond à environ 0,25 à 95 % du poids des microsphères et de préférence de 4 à 76 % dudit poids ; celle des charges fluorescentes éventuelles correspond à 0,01 à 0,5 % du poids desdites microsphères.

Les polysilsesquioxanes SiVi peuvent être obtenus par polycondensation d'un alcoxysilane de formule (I)

R-Si-(OR′)₃ (I)

ou d'un alcoxysiloxane de formule (II) 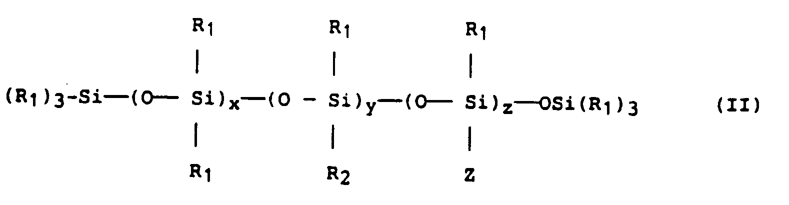
formules dans lesquelles
- R représente :
   . un radical vinyle
   . un radical éthyléniquement insaturé (de préférence ester insaturé tel que méthacryloxy-propyl)
- R₁ représente :
   . un radical alkyle en C₁-C₃ (de préférence méthyle ou éthyle)
   . un radical phényle
- R₂ représente :
   . un radical R₁
   . un radical alkyle en C₁-C₄ substitué par des groupes réactifs ou ionogènes tels que amino, époxy, mercapto, halogéno (de préférence aminopropyle, glycidylpropyle, mercatopropyle, bromopropyle, chloropropyle, trifluoropropyle)
   . un radical vinyle, le nombre de radicaux vinyles étant de deux au moins par molécule
- OR′ est un radical OH ou un radical hydrolysable tels que ceux dans lesquels R′ représente :
   . un radical alkyle en C₁-C₄
   . un radical -CO-CH₃, -CO-C₂H₅, -CO-CH₂-CO-CH₃, -CH₂-CH₂OH, -CH₂CH₂-OCH₃, -CH₂-CH₂-OC₂H₅
- Z représente un radical -r-Si(R₁)₃₋ₙ-(OR′)ₙ dans lequel r est un groupement alkylène en C₁-C₁₈, de préférence en C₂-C₆ et n est un nombre entier de 0 à 3
- Les symboles x, y et z ont une valeur suffisante pour assurer une viscosité inférieure à 100 mPas à 25° (de préférence de 10 à 100 mPas à 25° C), les symboles x et y pouvant être séparement nuls.

L'organohydrogénopolysiloxane SiH peut être linéaire, ramifié ou cyclique.

Parmi les organohydrogénopolysiloxanes SiH préférentiels on peut citer ceux de formule (III) :

Y (R₁)₂ SiO-(R₁ R˝ SiO)ₚ-(Y R₁ SiO)_{q}-Si (R₁)₂ Y (III)

formule dans laquelle les symboles R₁ sont identiques ou différents et ont la définition donnée ci-dessus, avec au moins 80 % de radicaux méthyle, les symboles Y représentent R₁ ou un atome d'hydrogène, le nombre d'atomes d'hydrogène étant de 3 au moins par molécule de polymère, les symboles R˝ représentent R₁ ou un radical alkyle en C₁ - C₄ substitué par des groupes réactifs ou ionogènes choisis parmi les motifs amino, époxy, mercapto, halogéno (de préférence aminopropyle, glycidylpropyle, mercaptopropyle, bromopropyle, chloropropyle, trifluoropropyle),les symboles p et q étant tels que ledit polymère SiH présente une viscosité de l'ordre de 5 à 1500 mPas à 25°C, de préférence de l'ordre de 20 à 150 mPas à 25°C et un nombre de motifs ionogènes et/ou réactifs non vinyliques éventuels allant de 1 à 1000, de préférence de l'ordre de 5 à 500 par molécule d'organohydrogénopolysiloxane SiH et de polysilsesquioxane SiVi.

Les polymères SiH ne portant pas de motifs ionogènes et/ou réactifs non vinyliques sont bien connus. Ils sont décrits par exemple dans les brevets américains n° 3 220 972 n° 3 344 111 et 3 436 366.

Les polymères SiH portant des groupes ionogènes et/ou réactifs non vinyliques peuvent être préparés selon des méthodes bien connues.

Les polymères SiH portant des groupes ionogènes et/ou réactifs non vinyliques peuvent être obtenus par exemple par :
- équilibrage d'un cyclotétrasiloxane et d'une huile polysiloxane à fonctions SiH internes en présence d'un disiloxane fonctionnalisé non réactif vis-à-vis des groupements SiH
- équilibrage d'un cyclotétrasiloxane fonctionnalisé (non réactif vis-à-vis de groupements SiH) en présence d'un dihydrogénodisiloxane
- équilibrage d'une huile polysiloxane fonctionnalisée (non réactive vis-à-vis de groupements SiH) en présence d'un dihydrogénodisiloxane ou d'un polysiloxane à fonctions SiH internes.

Pour une bonne réalisation de l'invention, le rapport en nombre des "groupes SiH" (atome d'hydrogène lié à un atome de silicium) sur les "groupes SiVi" (groupe éthyléniquement insaturé lié directement ou indirectement à un atome de silicium) soit compris entre 0,75/1 et 4/1, de préférence entre 0,75/1 et 1,5/1.

Le rapport pondéral organohydrogénopolysiloxane SiH/polysilsesquioxane SiVi peut aller de 5/100 à 100/100, de préférence de 5/100 à 30/100.

Les microsphères magnétisables faisant l'objet de l'invention sont parfaitement sphériques ; elles peuvent être de taille uniforme ou présenter une granulométrie étalée ; leur diamètre peut être de l'ordre de 0,05 à 3 micromètres et généralement de l'ordre de 0,2 à 2 micromètres.

Elles peuvent se présenter telles quelles ou en dispersion dans l'eau ; la quantité de microsphères magnétisables à l'état dispersé dans l'eau peut correspondre à environ 10 à 70 % en poids par rapport au poids total de dispersion et généralement de l'ordre de 15 à 50 % en poids.

Les microsphères composites magnétisables faisant l'objet de l'invention peuvent être préparées selon un procédé consistant à :
- à disperser dans un solvant organique non miscible à l'eau une suspension aqueuse de charges magnétisables non revêtues d'agent dispersant, charges présentant une taille généralement inférieure à 300x10⁻⁴ »m, de préférence de l'ordre de 50x10⁻⁴ à 120x10⁻⁴ »m,
- à dissoudre dans la phase organique de la dispersion obtenue un alcoxysilane ou un alcoxysiloxane de formules (I) ou (II) susceptible de se polycondenser en un polysesquioxane
- à polycondenser ledit alcoxysilane ou alcoxysiloxane
- à éliminer l'eau résultant de la polycondensation
- à dissoudre dans la phase organique de la dispersion obtenue au moins un organohydrogénopolysiloxane (appelé SiH) contenant par molécule au moins trois atomes d'hydrogène liés chacun à atome de silicium, présentant une viscosité de l'ordre de 5 à 1500 mPas à 25°C, de préférence entre 20 et 150 mPas à 25°C et portant éventuellement des motifs ionogènes et/ou réactifs non vinyliques choisis parmi les motifs amino, epoxy, mercapto et halogeno liés à un atome de silicium ou à un atome de carbone d'un groupement hydrocarboné relié à la chaîne organohydrogénopolysiloxane par une liaison Si-C, ainsi qu'un catalyseur d'hydrosilylation.
- à réticuler le mélange de polymères SiVi et SiH
- à séparer les microsphères magnétisables
- et éventuellement à redisperser lesdites particules dans de l'eau.

Une variante du procédé consiste à introduire tout ou partie de l'alcoxysilane dans la suspension aqueuse de charges magnétisables préalablement à la mise en dispersion de ladite suspension aqueuse dans le solvant organique.

Le solvant organique mis en oeuvre à l'étape de dispersion est un solvant des alcoxysilane ou des alcoxysiloxanes de formules (I) ou (II). A titre d'exemples, on peut citer le cyclohexane, le chlorure de méthylène, le benzène, l'hexane, le toluène, le tétrachlorure de carbone, l'octane, les esters de diacides gras.

L'opération de dispersion est réalisée en une ou plusieurs étapes à une température de l'ordre de 20 à 60°C, à l'aide d'un système d'agitation énergique tel que moulin à colloïdes, pompes à haute pression, agitateur à vibration, appareil à ultra-sons.

La suspension aqueuse de charges magnétisables peut être obtenue par mise en suspension de charges broyées ; toutefois une forme préférentielle de suspension est un sol aqueux de charges magnétisables obtenu par tout procédé connu comme par exemple celui décrit dans le brevet US n° 3.480.555.

La concentration de charges magnétisables dans la suspension aqueuse peut être de l'ordre de 0,5 à 50 % en poids, généralement de l'ordre de 5 à 20 % en poids. La quantité de charges mise en oeuvre est telle que le rapport pondéral charges magnétisables/alcoxysilane ou alcoxysiloxane soit de l'ordre de 0,005 à 50.

La quantité de solvant organique mise en oeuvre est telle que le rapport pondéral phase aqueuse/phase organique soit de l'ordre de 0,005 à 2.

Un agent tensio-actif est mis en oeuvre pour réaliser l'opération de dispersion. Celui-ci peut être choisi parmi ceux permettant d'obtenir des émulsions eau dans l'huile (de HLB généralement inférieur à 10, de préférence inférieur à 5) tels que les agents non-ioniques du type esters d'acides gras du sorbitol, mono et trioléates de sorbitan, copolymères séquencés oxyde d'éthylène/oxyde de propylène, les alkylphenols ethoxylés contenant moins de 10 motifs éthoxylés, les polycondensats d'acides gras, les copolymères séquencés organosiloxane-oxyde d'éthylène-oxyde de propylène ; les agents anioniques tels que les dialkylsulfosuccinates ; les agents cationiques tels que le bromure de cétylammonium, les copoly-condensats polyéthyléneimine-polyester.

L'opération de polycondensation est réalisée à une température de l'ordre de 20 à 80°C pendant environ 5 à 24 heures.

L'eau est ensuite éliminée par distillation par exemple.

On peut utiliser comme catalyseur de silylation des composés d'un métal du groupe du platine, en particulier leurs sels et complexes notamment l'acide chloroplatinique et les complexes de platine-oléfine comme décrit dans les brevets US-A 3 159 601 et 3 159 662, les produits de réaction des dérivés du platine avec des alcools, des aldéhydes et des éthers décrits dans le brevet US-A-3 220 972, les catalyseurs platine-vinylsiloxane décrits dans les brevets français FR-A 1 313 846 et son addition 88 676 et le brevet français FR-A 1 480 409 ainsi que les complexes décrits dans les brevets US-A 3 715 334, 3 775 452 et 3 814 730, ainsi qu'un catalyseur au rhodium tel que décrit dans les brevets US-A 3 296 291 et 3 928 629.

Les métaux préférés du groupe du platine sont le platine et le rhodium ; le ruthénium bien que moins actif mais moins cher, est également utilisable.

La quantité de catalyseur mise en oeuvre est généralement de l'ordre de 5 à 100 ppm, de préférence de 10 à 60 ppm, de catalyseur calculée en poids de métal par rapport au poids total des polymères SiVi et SiH.

Des exemples de polymères SiVi et SiH pouvant être utilisés ont déjà été cités ci-dessus. Pour une bonne réalisation de l'invention, les quantités relatives des deux types de polymère sont telles que le rapport en nombre des "groupes SiH" (atome d'hydrogène lié à un atome de silicium) sur les groupes "SiVi" (groupe éthyléniquement insaturé lié directement ou indirectement à un atome de silicium) soit compris entre 0,75/1 et 4/1, de préférence entre 0,75/1 et 1,5/1 et le rapport pondéral organohydrogénopolysiloxane SiH/polysilsesquioxane SiVi, soit compris entre 5/100 et 100/100, de préférence 5/100 et 30/100.

L'opération de réticulation peut être réalisée à une température de l'ordre de 20 à 90°C, de préférence de l'ordre de 50 à 70°C. Cette opération dure généralement 2 à 24 heures environ.

L'eau est ensuite éliminée par distillation par exemple.

Après refroidissement les microsphères magnétisables peuvent être séparées du milieu organique par tout moyen connu, notamment par aimantation.

Si désiré lesdites microsphères magnétisables peuvent être redispersées dans de l'eau déionisée jusqu'à obtenir un taux d'extrait sec de l'ordre de 10 à 70 % en poids, de préférence de l'ordre de 15 à 50 % en poids. Cette opération est réalisée en présence d'au moins un agent tensio-actif permettant d'obtenir des émulsions huile dans l'eau (de HBL généralement supérieur à 10, de préférence supérieur à 15) tel que alkylsulfates, alkylsulfonates.

Les microsphères magnétisables faisant l'objet de l'invention sont notamment intéressantes en biologie.

Elles peuvent être utilisées par exemple comme supports actifs :
. d'anticorps ou d'antigènes pour les tests de diagnostics, les séparations par affinité des composés biologiques ; la fixation des molécules biologiques peut, si nécessaire, être réalisée par des méthodes de couplage bien connues faisant intervenir des agents de couplage (glutaraldéhyde, carbodiimide hydrosoluble), ou bien consistant à activer les fonctions éventuelles du polyorganosiloxane (par exemple par diazotation, par action du bromure de cyanogène, de l'hydrazine) et à faire réagir la molécule à fixer
. de systèmes enzymatiques pour réactions biologiques
. de fixation de cultures cellulaires
. de médicaments ou de substances de révélation pour guider ceux-ci ou celles-ci in vitro ou in vivo vers le point de traitement choisi
. de molécules chimiques permettant une croissance de ces molécules par enchaînement rapide de réactions particulières comme la synthèse peptidique
. de groupements chimiques catalyseurs de réaction
. de groupements chimiques pour la séparation ou l'extraction de métaux ou d'isomères optiques.

Lesdites microsphères peuvent également être utilisées comme charges de renforcement pour élastomères ou pour la préparation de dispersions organiques utilisées dans les circuits hydrauliques de freins et d'amortisseurs.

Lorsqu'elles renferment en outre des charges luminescentes, elles peuvent être mises en oeuvre comme marqueur cellulaire ou comme agent de contraste en imagerie médicale.

La suspension aqueuse non surfactée d'oxyde de fer magnétique mise en oeuvre dans les exemples ci-après est préparée de la manière suivante :
175 g de Fe(NO3)3,9H20 et 75 g de Fe(SO4),7H₂O sont dissous dans 250 g d'eau permutée et 55 g d'acide nitrique concentré ; sous agitation rapide on ajoute 250 g d'une solution aqueuse à 20 % d'ammoniaque. Après décantation et élimination de la solution surnageante, le précipité est lavé une fois par de l'eau. Le milieu est ensuite ajusté à pH 0,5 par 35 g d'acide perchlorique, et le précipité filtré ; cette opération est répétée 3 fois, après quoi l'oxyde est remis en suspension dans de l'eau et ultrafiltré par de l'eau permutée. La suspension ainsi obtenue a une teneur en solide de 26,5 % pour un pH de 1,2. Le rendement exprimé Fe₃O₄ est de 57 %. L'examen en microscopie électronique à transmission indique des tailles de particules d'oxyde de fer comprise entre 50x10⁻⁴ et 200x10⁻⁴ micromètres.

### EXEMPLE 1 :

Préparation de microsphères magnétisables à base de poly(vinyl)silsesquioxane :

1,4 g de la suspension d'oxyde de fer préparée ci-dessus sont dilués par 0,6 g d'eau ; l'ensemble est dispersé dans un mélanae constitué de 50 g d'octane et 0,1 g de SPAN 80® (monooléate de sorbitan commercialisé par ICI (UK)), à l'aide d'un homogénéiseur ultrasonique. On introduit 2 g de vinyltrimethoxysilane (VTMO) à cette émulsion inverse qui est ensuite chargée dans un ballon verré de 100 ml que l'on adapte à un évaporateur rotatif ; le milieu est agité par rotation à une température de 50°C pendant 4 h puis une heure supplémentaire à 80°C pour chasser progressivement l'eau par distillation azéotropique. Les particules sont isolées par séparation magnétique et lavées par 30 g d'octane. La quantité de particules recueillie est de 1,25 g soit un rendement de polycondensation de 83 % (exprimé en poids de poly(vinyl)sil-sesqui-oxane formé).

La teneur en oxyde de fer dans les particules est de 30 % en poids, estimée par dosage du fer en absorption atomique ; les tailles de particules sont comprises entre 0,1 micromètre et 0,5 micromètre (mesurées en microscopie électronique à transmission).

### EXEMPLE 2 :

L'exemple 1 est répété à la différence que le vinyltriméthoxysilane (VTMO) est remplacé par le méthacryloxy-propyltriméthoxysilane (MEMO). Le rendement exprimé en poids de poly(méthacryloxy-propyl)silsesquioxane formé est de 42 %. La teneur en oxyde de fer dans les particules est de 47 %.

### EXEMPLE 3 :

Préparation de microsphères magnétisables "core-shell" composées d'un coeur Fe₃O₄/Poly(vinyl)silsesquioxane et d'une écorce polydiméthylsiloxane réticulé :

Les microsphères préparées dans l'exemple 1 sont dispersées dans 30 g d'octane en présence de 0,1 g de SPAN 80® ; on ajoute alors 2 g d'une huile organosiliciée hydrosilylée de formule :

R˝R′RSiO-(SiRR‴O)ₙ-(SiR‴R′O)ₘ-SiRR′R˝,

où : R=R′=R‴=-CH3, R˝=-CH:CH2
avec n+m=142
ainsi que 2 gouttes de catalyseur au platine (complexe Pt(divinyltetramethyl-disiloxane)₂).

L'ensemble est ensuite chargé dans un ballon verré de 100 ml que l'on adapte à un évaporateur rotatif ; le milieu est agité par rotation, à une température de 50° C pendant 3 heures. Les particules sont alors récupérées par décantation magnétique et dispersées dans un mélange eau-acétone 90-10 en présence de Cemulsol NP 30® (nonyl-phénol-éthoxylé à 30 molécules d'oxyde d'éthylène, commercialisé par SFOS (France)) à la concentration de 1 g/l ; l'octane résiduel est chassé par distillation azéotropique. La quantité de particules recueillies est de 2,4 g soit un rendement d'hydrosilylation de 57 % (exprime en poids de polydimethylsiloxane réticulé à la surface des particules). La teneur en oxyde de fer dans les particules est de 17 % en poids, les tailles des particules core-shell sont comprises entre 0,2 micromètre et 0,8 micromètre (mesurées en microscopie électronique à transmission).

### EXEMPLE 4 :

Préparation de microsphères magnétisables "core-shell " composées d'un coeur Fe₃O₄/Poly(méthacryloxy-propyl)silsesquioxane et d'une écorce polydiméthylsiloxane réticulé

On reprend les conditions de l'exemple 3, à partir des microsphères préparées selon l'exemple 2. Le rendement d'hydrosilylation est de 60 % (exprimé en poids de polydiméthylsiloxane réticulé à la surface des particules). La teneur en oxyde de fer dans les particules est de 16 % en poids.

### EXEMPLE 5 :

Préparation de microsphères magnétisables "core-shell" composées d'un coeur Fe₃O₄/Poly(vinyl)silsesquioxane et d'une écorce polydiméthylsiloxane réticulé fonctionnalisée époxy

On reprend les conditions de l'exemple 3, à partir des microsphères préparées selon l'exemple 1, excepté que l'huile organosiliciée hydrosilylée est remplacée par une huile organosilicié hydrosilylée et époxydée de formule :

R′₃SiO-(SiRR˝O)ₒ(SiRR‴O)ₚ-(SiYRO)_{q}-SiR′₃,

où R=R′=R˝=-CH3, Y=H, R‴=éther de glycidyl
avec o=33, p=6, q=6

Le rendement d'hydrosilylation est de 33 % (exprimé en poids de polymethylsiloxane époxydé réticulé à la surface des particules). La teneur en oxyde de fer dans les particules est de 20%.

### EXEMPLE 6 :

Préparation de microsphères magnétisables "core-shell" composées d'un coeur Fe₃O₄/Poly(vinyl)silsesquioxane et d'une écorce polydiméthylsiloxane réticulé fonctionnalisée amine

On reprend 1 g de microsphères préparées dans l'exemple 5 que l'on redisperse dans 25 g de toluene. La dispersion est ensuite chargée dans un réacteur verré de 100 ml thermorégulé muni d'une agitation et d'un condenseur. La température est amenée à 100° C ; on verse alors au goutte à goutte 0,8g d'une diamine alkoxylée de formule :

H₂N-CH(CH₃)CH₂-[O-CH(CH₃)CH2]ₐ-[OCH2CH2]_{b}-[O-CH(CH3)CH2]_{c}-NH2

avec a+c=2,5 et b=8,5 (Jeffamine® commercialisée par Texaco (USA).
Le milieu réactionnel est laissé à cette température pendant 15 heures. Après refroidissement, l'excès d'amine est éliminé par décantation magnétique. Les microsphères aminées sont ensuite redispersées dans l'eau pour obtenir un latex magnétisable.

### EXEMPLE 7 : (comparatif à l'exemple 1)

Dans cet exemple on substitue la suspension aqueuse non surfactée d'oxyde de fer décrite ci-dessus par une suspension d'oxyde de fer surfactée préparée selon le procédé revendiqué dans le brevet US 4094804 ; il s'agit d'oxyde de fer précipité en présence d'acide oleique, que l'on repeptise en milieu aqueux par ajout d'émulsifiant anionique (dioctylsulfosuccinate, Aerosol OT® commercialisé par American Cyanamid). La synthèse est poursuivie comme indiqué dans l'exemple 1 : dans ce cas on n'obtient pas de microsphères magnétisables ; en effet l'oxyde de fer a diffusé progressivement de la phase aqueuse vers la phase organique.

## Revendications (Revendications pour l'(les) Etat(s) contractant(s) suivant(s): AT, BE, CH, DE, DK, FR, GB, GR, IT, LI, LU, NL, SE)

1. Microsphères composites magnétisables à base d'un polymère organosilicié réticulé se présentant telles quelles ou en dispersion aqueuse, caractérisées en ce qu'elles sont formées :
- d'un coeur constitué de charges magnétisables présentant une taille généralement inférieure à 300x10⁻⁴ »m, de préférence de l'ordre de 50 à 120x10⁻⁴ »m, réparties uniformément à l'intérieur d'un réseau de polysilsesquioxane (appelé SiVi) contenant par molécule au moins deux groupes éthyleniquement insaturés ("groupe SiVi") liés chacun à un atome de silicium ou à un atome de carbone d'un groupement organique relié à la chaîne polysilsesquioxane par une liaison Si-C, portant éventuellement des motifs ionogènes et/ou réactifs non éthyléniques, choisis parmi les motifs amino, époxy, mercapto et halogeno, liés à un atome de silicium ou à un atome de carbone d'un groupement hydrocarboné relié à la chaîne polysilsesquioxane par une liaison Si-C
- et d'une écorce à base d'un polymère organosilicié réticulé dérivé de l'hydrosilylation
. d'un organohydrogénopolysiloxane (appelé SiH) contenant par molécule au moins trois atomes d'hydrogène liés chacun à un atome de silicium ("groupes SiH"), présentant une viscosité de l'ordre de 5 à 1500 mPas à 25°C et portant éventuellement des motifs ionogènes et/ou réactifs non vinyliques, choisis parmi les motifs amino, époxy, mercapto et halogeno, liés à un atome de silicium ou à un atome de carbone d'un groupement hydrocarboné relié à la chaîne organohydrogénopolysiloxane par une liaison Si-C
. avec les groupes éthyléniquement insaturés dudit polysilsesquioxane SiVi.

2. Microsphères selon la revendication 1 caractérisées en ce que la quantité de charges magnétisables correspond à environ 0,25 à 95 % du poids des microsphères.

3. Microsphères selon la revendication 1 ou 2 caractérisées en ce que le rapport en nombre des "groupes SiH" aux "groupes SiVi" est de l'ordre de 0,75/1 à 4/1.

4. Microsphères selon l'une quelconque des revendications 1 à 4, caractérisées en ce que le rapport pondéral organohydrogénopolysiloxane SiH/polysilsesquioxane SiVi est de l'ordre de 5/100 à 100/100.

5. Microsphères selon l'une quelconque des revendications 1 à 4, caractérisées en ce que le polysilsesquioxane SiVi dérive de la polycondensation d'un alcoxysilane de formule (I)
R-Si-(OR′)₃ (I)
ou d'un alcoxysiloxane de formule (II) 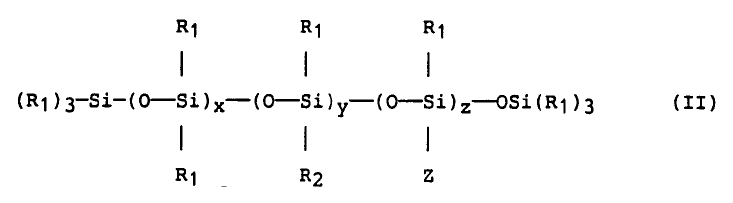 formules dans lesquelles
- R représente :
. un radical vinyle
. un radical éthyléniquement insaturé tel que méthacryloxy-propyl
- R₁ représente :
. un radical alkyle en C₁-C₃
. un radical phényle
- R₂ représente :
. un radical R₁
. un radical alkyle en C₁-C₄ substitué par des groupes réactifs ou ionogènes tels que amino, époxy, mercapto, halogéno
. un radical vinyle, le nombre de radicaux vinyles étant de deux au moins par molécule
- OR′ est un radical OH ou un radical hydrolysable tels que ceux dans lesquels R′ représente :
. un radical alkyle en C₁-C₄
. un radical -CO-CH₃, -CO-C₂H₅, -CO-CH₂-CO-CH₃, -CH₂-CH₂OH, -CH₂CH₂-OCH₃, -CH₂-CH₂-OC₂H₅
- Z représente un radical -r-Si(R₁)₃₋ₙ-(OR′)ₙ dans lequel r est un groupement alkylène en C₁-C₁₈ et n est un nombre entier de 0 à 3
- Les symboles x, y et z ont une valeur suffisante pour assurer une viscosité inférieure à 100 mPas à 25°, les symboles x et y pouvant être séparement nuls.

6. Microsphères selon l'une quelconque des revendications 1 à 5, caractérisées en ce que l'organohydrogénopolysiloxane SiH a pour formule :
Y (R₁)₂ SiO-(R₁ R˝ SiO)ₚ-(Y R₁ SiO)_{q}-Si (R₁)₂ Y (III)
dans laquelle les symboles R₁ sont identiques ou différents et ont la définition donnée dans la revendication 5, avec au moins 80 % de radicaux méthyle, les symboles Y représentent R₁ ou un atome d'hydrogène, le nombre d'atomes d'hydrogène étant de 3 au moins par molécule de polymère, les symboles R˝ représentent R₁ ou un radical alkyle en C₁ - C₄ substitué par des groupes réactifs ou ionogènes choisis parmi les motifs amino, époxy, mercapto, halogéno, les symboles p et q étant tels que ledit polymère SiH présente une viscosité de l'ordre de 5 à 1500 mPas à 25°C, et un nombre de motifs ionogènes et/ou réactifs non vinyliques éventuels allant de 1 à 1000 par molécule d'organohydrogénopolysiloxane SiH et de polysilsesquioxane SiVi.

7. Microsphères selon l'une quelconque des revendications 1 à 6, caractérisées en ce qu'elles présentent une granulométrie de l'ordre de 0,05 à 3 micromètres.

8. Procédé de préparation de microsphères composites consistant :
- à disperser dans un solvant organique non miscible à l'eau une suspension aqueuse de charges magnétisables non revêtues d'agent dispersant, charges présentant une taille généralement inférieure à 300x10⁻⁴ »m, de préférence de l'ordre de 50x10⁻⁴ à 120x10⁻⁴ »m,
- à dissoudre dans la phase organique de la dispersion obtenue un alcoxysilane ou un alcoxysiloxane susceptible de se polycondenser en un polysilsesquioxane (SiVi) contenant par molécule au moins deux groupes éthyléniquement insaturés ("groupe SiVi") liés chacun à un atome de silicium ou à un atome de carbone d'un groupement organique relié à la chaîne polysilsesquioxane par une liaison SiC, portant éventuellement des motifs ionogènes et/ou réactifs non éthyléniques choisis parmi les motifs amino, époxy, mercapto et halogeno liés à un atome de silicium ou à un atome de carbone d'un groupement hydrocarboné relié à la chaîne polysilsesquioxane par une liaison Si-C,
- à polycondenser ledit alcoxysilane ou alcoxysiloxane en un polysilsesquioxane SiVi,
- à éliminer l'eau résultant de la polycondensation,
- à dissoudre dans la phase organique de la dispersion obtenue au moins un organohydrogénopolysiloxane (appelé SiH) contenant par molécule au moins trois atomes d'hydrogène liés chacun à atome de silicium, présentant une viscosité de l'ordre de 5 à 1500 mPas à 25°C et portant éventuellement des motifs ionogènes et/ou réactifs non vinyliques, choisis parmi les motifs amino, époxy, mercapto et halogeno liés à un atome de silicium ou à un atome de carbone d'un groupement hydrocarboné relié à la chaîne organohydrogénopolysiloxane par une liaison Si-C, ainsi qu'un catalyseur d'hydrosilylation.
- à réticuler le mélange de polymères SiVi et SiH,
- à séparer les microsphères magnétisables,
- et éventuellement à redisperser lesdites particules dans de l'eau.

9. Procédé selon la revendication 8. caractérisé en ce que la concentration de charges magnétisables dans la suspension aqueuse est de l'ordre de 0,5 à 50 % en poids.

10. Procédé selon la revendication 8 ou 9, caractérisé en ce que la quantité de charges mise en oeuvre est telle que le rapport pondéral charges magnétisables/alcoxysilane ou alcoxysiloxane est de l'ordre de 0,005 à 50.

11. Procédé selon l'une quelconque des revendications 8 à 10, caractérisé en ce que le rapport pondéral phase aqueuse/phase organique est de l'ordre de 0,005 à 2.

12. Procédé selon l'une quelconque des revendications 8 à 11 caractérisé en ce que l'alcoxysilane a pour formule (I) :
R-Si-(OR′)₃ (I)
et l'alcoxysiloxane a pour formule (II) : 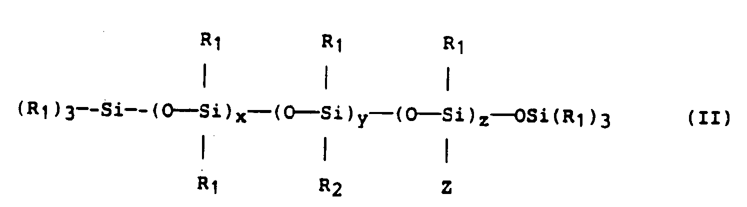 dans lesquelles
- R représente :
. un radical vinyle
. un radical éthyléniquement insaturé tel que méthacryloxy propyl
- R₁ représente :
. un radical alkyle en C₁-C₃
. un radical phényle
- R₂ représente :
. un radical R₁
. un radical alkyle en C₁-C₄ substitué par des groupes réactifs ou ionogènes tels que amino, époxy, mercapto, halogéno
. un radical vinyle, le nombre de radicaux vinyles étant de deux au moins par molécule
- OR′ est un radical OH ou un radical hydrolysable tels que ceux dans lesquels R′ représente :
. un radical alkyle en C₁-C₄
. un radical -CO-CH₃, -CO-C₂H₅, -CO-CH₂-CO-CH₃, -CH₂-CH₂OH, -CH₂CH₂-OCH₃, -CH₂-CH₂-OC₂H₅
- Z représente un radical -r-Si(R₁)₃₋ₙ-(OR′)ₙ dans lequel r est un groupement alkylène en C₁-C₁₈ et n est un nombre entier de 0 à 3
- Les symboles x, y et z ont une valeur suffisante pour assurer une viscosité inférieure à 100 mPas à 25°, les symboles x et y pouvant être séparement nuls.

13. Procédé selon l'une quelconque des revendications 8 à 12, caractérisé en ce que l'organohydrogénopolysiloxane SiH a pour formule :
Y (R₁)₂ SiO-(R₁ R˝ SiO)ₚ-(Y R₁ SiO)_{q}-Si (R₁)₂ Y (III)
dans laquelle les symboles R₁ sont identiques ou différents et ont la définition donnée dans la revendication 12, avec au moins 80 % de radicaux méthyle, les symboles Y représentent R₁ ou un atome d'hydrogène, le nombre d'atomes d'hydrogène étant de 3 au moins par molécule de polymère, les symboles R˝ représentent R₁ ou un radical alkyle en C₁ - C₄ substitué par des groupes réactifs ou ionogènes choisis parmi les motifs amino, époxy, mercapto, halogéno, les symboles p et q étant tels que ledit polymère SiH présente une viscosité de l'ordre de 5 à 1500 mPas à 25°C, et un nombre de motifs ionogènes et/ou réactifs non vinyliques éventuels allant de 1 à 1000 par molécule d'organohydrogénopolysiloxane SiH et de polysilsesquioxane SiVi.

14. Procédé selon l'une quelconque des revendications 8 à 13, caractérisé en ce que le rapport pondéral organohydrogénopolysiloxane SiH/polysilsesquioxane SiVi est de l'ordre de 5/100 à 100/100.

15. Procédé selon l'une quelconque des revendications 8 à 14, caractérisé en ce que le rapport en nombre "groupes SiH" aux "groupes SiVi" est de l'ordre de 0,75/1 à 4/1.

16. Utilisation des microsphères telles quelles ou en dispersion aqueuse faisant l'objet de l'une quelconque des revendications 1 à 7 ou obtenues selon le procédé faisant l'objet de l'une quelconque des revendications 8 à 15 comme supports actifs en biologie, à condition que les méthodes de traitement thérapeutique telles que définies à l'article 52 (4) CEB soient exclues.

## Revendications (Revendications pour l'(les) Etat(s) contractant(s) suivant(s): ES)

1. Procédé de préparation de microsphères composites consistant :
- à disperser dans un solvant organique non miscible à l'eau une suspension aqueuse de charges magnétisables non revêtues d'agent dispersant, charges présentant une taille généralement inférieure à 300x10⁻⁴ »m, de préférence de l'ordre de 50x10⁻⁴ à 120x10⁻⁴ »m,
- à dissoudre dans la phase organique de la dispersion obtenue un alcoxysilane ou un alcoxysiloxane susceptible de se polycondenser en un polysilsesquioxane (SiVi) contenant par molécule au moins deux groupes éthyléniquement insaturés ("groupe SiVi") liés chacun à un atome de silicium ou à un atome de carbone d'un groupement organique relié à la chaîne polysilsesquioxane par une liaison SiC, portant éventuellement des motifs ionogènes et/ou réactifs non éthyléniques choisis parmi les motifs amino, époxy, mercapto et halogéno liés à un atome de silicium ou à un atome de carbone d'un groupement hydrocarboné relié à la chaîne polysilsesquioxane par une liaison Si-C,
- à polycondenser ledit alcoxysilane ou alcoxysiloxane en un polysilsesquioxane SiVi,
- à éliminer l'eau résultant de la polycondensation,
- à dissoudre dans la phase organique de la dispersion obtenue au moins un organohydrogénopolysiloxane (appelé SiH) contenant par molécule au moins trois atomes d'hydrogène liés chacun à atome de silicium, présentant une viscosité de l'ordre de 5 à 1500 mPas à 25°C et portant éventuellement des motifs ionogènes et/ou réactifs non vinyliques choisis parmi les motifs amino, époxy, mercapto et halogéno liés à un atome de silicium ou à un atome de carbone d'un groupement hydrocarboné relié à la chaîne organohydrogénopolysiloxane par une liaison Si-C, ainsi qu'un catalyseur d'hydrosilylation.
- à réticuler le mélange de polymères SiVi et SiH,
- à séparer les microsphères magnétisables,
- et éventuellement à redisperser lesdites particules dans de l'eau.

2. Procédé selon la revendication 1. caractérisé en ce que la concentration de charges magnétisables dans la suspension aqueuse est de l'ordre de 0,5 à 50 % en poids.

3. Procédé selon la revendication 1 ou 2, caractérisé en ce que la quantité de charges mise en oeuvre est telle que le rapport pondéral charges magnétisables/alcoxysilane ou alcoxysiloxane est de l'ordre de 0,005 à 50.

4. Procédé selon l'une quelconque des revendications 1 à 3, caractérisé en ce que le rapport pondéral phase aqueuse/phase organique est de l'ordre de 0,005 à 2.

5. Procédé selon l'une quelconque des revendications 1 à 4 caractérisé en ce que l'alcoxysilane a pour formule (I) :
R-Si-(OR′)₃ (I)
et l'alcoxysiloxane a pour formule (II) : 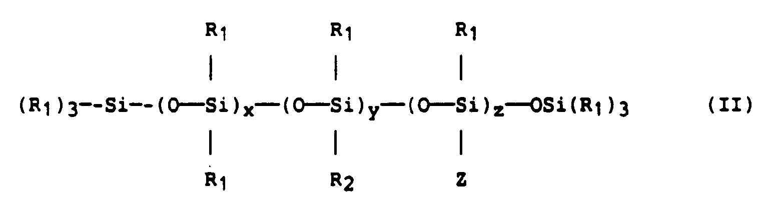 dans lesquelles
- R représente :
. un radical vinyle
. un radical éthyléniquement insaturé tel que méthacryloxy propyl
- R₁ représente :
. un radical alkyle en C₁-C₃
. un radical phényle
- R₂ représente :
. un radical R₁
. un radical alkyle en C₁-C₄ substitué par des groupes réactifs ou ionogènes tels que amino, époxy, mercapto, halogéno
. un radical vinyle, le nombre de radicaux vinyles étant de deux au moins par molécule
- OR′ est un radical OH ou un radical hydrolysable tels que ceux dans lesquels R′ représente :
. un radical alkyle en C₁-C₄
. un radical -CO-CH₃, -CO-C₂H₅, -CO-CH₂-CO-CH₃, -CH₂-CH₂OH, -CH₂CH₂-OCH₃, -CH₂-CH₂-OC₂H₅
- Z représente un radical -r-Si(R₁)₃₋ₙ-(OR′)ₙ dans lequel r est un groupement alkylène en C₁-C₁₈ et n est un nombre entier de 0 à 3
- Les symboles x, y et z ont une valeur suffisante pour assurer une viscosité inférieure à 100 mPas à 25°, les symboles x et y pouvant être séparement nuls.

6. Procédé selon l'une quelconque des revendications 1 à 5, caractérisé en ce que l'organohydrogénopolysiloxane SiH a pour formule :
Y (R₁)₂ SiO-(R₁ R˝ SiO)ₚ-(Y R₁ SiO)_{q}-Si (R₁)₂ Y (III)
dans laquelle les symboles R₁ sont identiques ou différents et ont la définition donnée dans la revendication 12, avec au moins 80 % de radicaux méthyle, les symboles Y représentent R₁ ou un atome d'hydrogène, le nombre d'atomes d'hydrogène étant de 3 au moins par molécule de polymère, les symboles R˝ représentent R₁ ou un radical alkyle en C₁ - C₄ substitué par des groupes réactifs ou ionogènes choisis parmi les motifs amino, époxy, mercapto, halogéno, les symboles p et q étant tels que ledit polymère SiH présente une viscosité de l'ordre de 5 à 1500 mPas à 25°C, et un nombre de motifs ionogènes et/ou réactifs non vinyliques éventuels allant de 1 à 1000 par molécule d'organohydrogénopolysiloxane SiH et de polysilsesquioxane SiVi.

7. Procédé selon l'une quelconque des revendications 1 à 6, caractérisé en ce que le rapport pondéral organohydrogénopolysiloxane SiH/polysilsesquioxane SiVi est de l'ordre de 5/100 à 100/100.

8. Procédé selon l'une quelconque des revendications 1 à 7, caractérisé en ce que le rapport en nombre "groupes SiH" aux "groupes SiVi" est de l'ordre de 0,75/1 à 4/1.

9. Utilisation des microsphères telles quelles ou en dispersion aqueuse obtenues selon le procédé faisant l'objet de l'une quelconque des revendications 1 à 8 comme supports actifs en biologie, à condition que les méthodes de traitement thérapeutique telles que définies à l'article 52(4) CEB soient exclues.

## Patentansprüche (Patentansprüche für folgende(n) Vertragsstaat(en): AT, BE, CH, DE, DK, FR, GB, GR, IT, LI, LU, NL, SE)

1. Magnetisierbare Mikroverbundkugeln auf der Basis eines vernetzten Organosiliziumpolymers, die als solche oder in wässriger Dispersion vorliegen, dadurch gekennzeichnet, daß sie gebildet sind aus:
- einem Kern bestehend aus magnetisierbaren Füllungen, die im allgemeinen eine Größe kleiner als 300x10⁻⁴ »m, bevorzugt im Bereich von 50 bis 120x10⁻⁴ »m haben, gleichmäßig im Inneren eines Netzes aus Polysilasesquioxan (genannt SiVi) verteilt, das pro Molekül mindestens zwei ethylenisch ungesättigte Gruppen ("Gruppe SiVi") enthält,jeweils gebunden an ein Siliziumatom oder an ein Kohlenstoffatom einer über eine Si-C-Bindung mit der Polysilasesquioxankette verbundenen organischen Gruppe, das weiter gegebenenfalls ionogene und/oder reaktive, nicht ethylenische Gruppen trägt, ausgewählt aus den Gruppen Amino, Epoxy, Mercapto und Halogen, gebunden an ein Siliziumatom oder an ein Kohlenstoffatom einer über eine Si-C-Bindung an die Polysilasesquioxankette gebundenen Kohlenwasserstoffgruppe
- und aus einer Schale auf der Basis eines vernetzten Organosiliziumpolymeren, erhalten aus der Hydrosilylierung
. eines Organopolysilans (Organohydrogenpolysiloxans) (genannt SiH), das pro Molekül mindestens drei Wasserstoffatome enthält, jedes an ein Siliziumatom gebunden ("SiH-Gruppe"), das eine Viskosität im Bereich von 5 bis 1500 mPas bei 25°C aufweist und das gegebenenfalls ionogene und/oder reaktive, nicht-vinylische Gruppen, ausgewählt aus den Gruppen Amino, Epoxy, Mercapto und Halogen trägt, gebunden an ein Siliziumatom oder an ein Kohlenstoffatom einer über eine Si-C-Bindung an die Organopolysilangruppe (Organohydrogenpolysiloxangruppe) gebundenen Kohlenwasserstoffgruppe,
. mit ethylenisch ungesättigten Gruppen des genannten Polysilasesquioxans SiVi.

2. Mikrokugeln nach Anspruch 1, dadurch gekennzeichnet, daß die Menge der magnetisierbaren Füllungen ungefähr 0,25 bis 95 Gew.% der Mikrokugeln entspricht.

3. Mikrokugeln nach Anspruch 1 oder 2, dadurch gekennzeichnet, daß das Zahlenverhältnis der "SiH-Gruppen" zu den "SiVi-Gruppen" im Bereich von 0,75/1 bis 4/1 liegt.

4. Mikrokugeln nach einem der Ansprüche 1 bis 4, dadurch gekennzeichnet, daß das Gewichtsverhältnis Organopolysilan (Organohydrogenpolysiloxan) SiH/Polysilasesquioxan SiVi in dem Bereich von 5/100 bis 100/100 liegt.

5. Mikrokugeln nach einem der Ansprüche 1 bis 4, dadurch gekennzeichnet, daß das Polysilasesquioxan SiVi erhalten wird durch Polykondensation eines Alkoxysilans der Formel (I)
R-Si-(OR′)₃ (I)
oder eines Alkoxysiloxans der Formel (II) 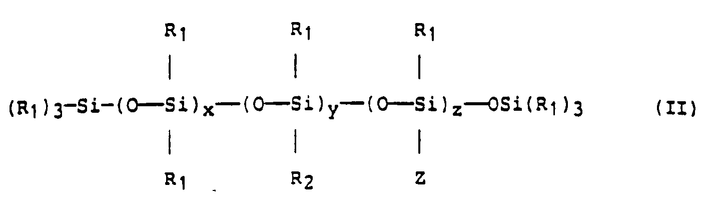 wobei in den Formeln
- R darstellt:
. einen Vinylrest
. einen ethylenisch ungesättigten Rest wie Methylacryloxypropyl
- R₁ darstellt:
. einen C₁-C₃-Alkylrest (bevorzugt Methyl oder Ethyl)
. einen Phenylrest
- R₂ bedeutet:
. einen R₁-Rest
. einen C₁-C₄-Alkylrest, substituiert mit reaktiven oder ionogenen Gruppen wie Amino-, Epoxy-, Mercapto-, Halogen-
. einen Vinylrest, wobei die Anzahl der Vinylgruppen mindestens zwei pro Mokekül beträgt,
- OR′ ein OH-Rest ist oder ein hydrolysierbarer Rest wie solche, worin R′ bedeutet:
. einen C₁-C₄-Alkylrest
. einen -CO-CH₃-, -CO-C₂H₅-, -CO-CH₂-CO-CH₃-, -CH₂-CH₂OH-, -CH₂CH₂-OCH₃-, -CH₂-CH₂-OC₂H₅-Rest
- Z einen Rest -r-Si(R₁)-₃₋ₙ-(OR′)ₙ bedeutet, worin r eine C₁-C₁₈-Alkylengruppe ist, und n eine ganze Zahl von 0 bis 3 ist;
- die Symbole x, y, z einen ausreichenden Wert haben, um eine Viskosität niedriger als 100 mPas bei 25°C zu gewährleisten, wobei die Symbole x und y jeweils unabhängig voneinander Null sein können.

6. Mikrokugeln nach einem der Ansprüche 1 bis 5, dadurch gekennzeichnet, daß das Organopolysilan (Organohydrogenpolysiloxan) SiH die Formel
Y (R₁)₂ SiO-(R₁ R˝ SiO)ₚ-(Y R₁ SiO)_{q}-Si (R₁)₂ Y (III)
hat, in welcher die Symbole R₁ gleich oder verschieden sind und wie in Anspruch 5 definiert sind, mit mindestens 80% Methylresten, die Symbole y R₁ oder ein Wasserstoffatom darstellen, wobei die Zahl der Wasserstoffatome mindestens 3 pro Polymermolekül ist, die Symbole R" R₁ oder einen C₁-C₄-Alkylrest, substituiert mit reaktiven oder ionogenen Gruppen, ausgewählt aus den Resten Amino, Epoxy, Mercapto, Halogen darstellen, wobei die Symbole p und q solche sind, daß das genannte Polymer SiH eine Viskosität im Bereich von 5 bis 1500 mPas bei 25°C aufweist, und eine Zahl der ionogenen und/oder reaktiven, nicht-vinylischen Gruppen gegebenenfalls in dem Bereich von 1 bis 1000 pro Organopolysilanmolekül (Organohydrogenpolysiloxan) SiH und Polysilasesquioxan SiVi liegt.

7. Mikrokugeln nach einem der Ansprüche 1 bis 6, dadurch gekennzeichnet, daß sie eine Teilchengrößenanalyse (Granulometrie) im Bereich von 0,05 bis 3 Mikrometern aufweisen.

8. Verfahren zur Herstellung von Mikroverbundkugeln, welches besteht aus:
- Dispergieren einer wässrigen Suspension von magnetisierbaren, nicht mit Dispergiermittel umhüllten Füllungen in einem organischen, nicht mit Wasser mischbaren Lösungsmittel, wobei die Füllungen im allgemeinen eine Größe kleiner 300x10⁻⁴ »m, bevorzugt im Bereich von 50x10⁻⁴ bis 120x10⁻⁴ » aufweisen,
- Lösen eines zu einem Polysilasesquioxan (SiVi) polykondensierbaren Alkoxysilans oder Alkoxysiloxans, welches pro Molekül mindestens zwei ethylenisch ungesättigte Gruppen ("SiVi-Gruppe") trägt, jede gebunden an ein Siliziumatom oder an ein Kohlenstoffatom einer über eine SiC-Bindung an die Polysilasesquioxangruppe gebundenen organischen Gruppe, gegebenenfalls ionogene und/oder reaktive, nicht-ethylenische Gruppen trägt, ausgewählt aus den Amino-, Epoxy- Mercapto- und Halogenresten, gebunden an ein Siliziumatom oder an ein Kohlenstoffatom einer über eine Si-C-Bindung an die Polysilasesquioxankette gebundenen Kohlenwasserstoffgruppe, in der organischen Phase der erhaltenen Suspension,
- Polykondensieren des genannten Alkoxysilans oder Alkoxysiloxans zu einem Polysilasesquioxan SiVi,
- Eliminieren des bei der Polykondensation erhaltenen Wassers,
- Lösen von mindestens einem Organopolysilan (Organohydrogenpolysiloxan) (genannt SiH), das pro Molekül mindestens drei Wasserstoffatome enthält, jedes gebunden an ein Siliziumatom, das eine Viskosität im Bereich von 5 bis 1500 mPas bei 25°C aufweist und gegebenenfalls ionogene und/oder reaktive, nicht-vinylische Gruppen trägt, ausgewählt aus den Gruppen Amino, Epoxy, Mercapto und Halogen, gebunden an ein Siliziumatom oder ein Kohlenstoffatom einer über eine Si-C-Bindung an die Organopolysilankette (Organohydrogenpolysiloxankette) gebundenen Kohlenwasserstoffgruppe, sowie eines Hydrosilylierkatalysators in der organische Phase der erhaltenen Dispersion
- Vernetzen der Mischung aus den Polymeren SiVi und SiH,
- Abtrennen der magnetisierbaren Mikrokugeln,
- und gegebenenfalls erneutem Dispergieren der genannten Teilchen in Wasser.

9. Verfahren nach Anspruch 8, dadurch gekennzeichnet, daß die Konzentration der magnetisierbaren Füllungen in der wässrigen Suspension in dem Bereich von 0,5 bis 50 Gew. % ist.

10. Verfahren nach Anspruch 8 oder 9, dadurch gekennzeichnet, daß die Menge der verwendeten Füllungen so ist, daß das Gewichtsverhältnis von magnetisierbaren Füllungen/Alkoxysilan oder Alkoxysiloxan im Bereich von 0,005 bis 50 ist.

11. Verfahren nach einem der Ansprüche 8 bis 10, dadurch gekennzeichnet, daß das Gewichtsverhältnis wässrige Phase/organische Phase in dem Bereich von 0,005 bis 2 ist.

12. Verfahren nach einem der Ansprüche 8 bis 11, dadurch gekennzeichnet, daß das Alkoxysilan die Formel (I):
R-Si-(OR′)₃ (I)
hat oder das Alkoxysiloxan die Formel (II) 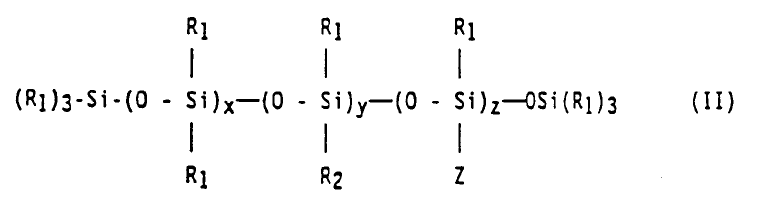 hat, wobei in den Formeln
- R bedeutet:
. einen Vinylrest
. einen ethylenisch ungesättigten Resten wie Methacryloxypropyl
- R₁ bedeutet:
. einen C₁-C₃-Alkylrest
. einen Phenylrest
- R₂ bedeutet:
. einen R₁-Rest
. einen C₁-C₄-Alkylrest, substituiert mit reaktiven oder ionogenen Gruppen wie Amino-, Epoxy-, Mercapto-, Halogen
. einen Vinylrest, wobei die Anzahl der Vinylreste im Molkekül mindestens zwei beträgt,
- OR′ ein OH-Rest oder ein hydrolysierbarer Rest ist wie solche, worin R′ bedeutet:
. einen C₁-C₄-Alkylrest
. einen -CO-CH₃-, -CO-C₂H₅-, -CO-CH₂-CO-CH₃-, -CH₂-CH₂OH-, -CH₂CH₂-OCH₃-, -CH₂-CH₂-OC₂H₅-Rest
- Z einen -r-Si(R₁)-₃₋ₙ₋(OR′)ₙ Rest bedeutet, worin r eine C₁-C₁₈-Alkylengruppe ist, und n eine ganze Zahl von 0 bis 3 ist;
- die Symbole x, y, z einen ausreichenden Wert haben, um eine Viskosität niedriger als 100 mPas bei 25°C zu gewährleisten, wobei die Symbole x und y jeweils unabhängig voneinander Null sein können.

13. Verfahren nach einem der Ansprüche 8 bis 12, dadurch gekennzeichnet, daß das Organopolysilan (Organohydrogenpolysiloxan) SiH die Formel
Y (R₁)₂ SiO-(R₁ R˝ SiO)ₚ-(Y R₁ SiO)_{q}-Si (R₁)₂ Y (III)
hat, in welcher die Symbole R₁ gleich oder verschieden sind und wie in Anspruch 12 definiert sind, mit mindestens 80% Methylresten, die Symbole y R¹ oder ein Wasserstoffatom darstellen, wobei die Zahl der Wasserstoffatome mindestens 3 pro Polymermolekül ist, die Symbole R" R₁ oder einen C₁-C₄-Alkylrest substituiert mit reaktiven oder ionogenen Gruppen ausgewählt aus den Resten Amino, Epoxy, Mercapto, Halogen, darstellen, wobei die Symbole p und q solche sind, daß das genannte Polymer SiH eine Viskosität im Bereich von 5 bis 1500 mPas bei 25°C aufweist, und eine Zahl der ionogenen und/oder reaktiven, nicht-vinylischen Gruppen gegebenenfalls im Bereich von 1 bis 1000 pro Organopolysilanmolekül (Organohydrogenpolysiloxanmolekül) SiH und Polysilasesquioxanmolekül SiVi liegt.

14. Verfahren nach einem der Ansprüche 8 bis 13, dadurch gekennzeichnet, daß das Gewichtsverhältnis von Organopolysilan (Organohydrogenpolysiloxan) SiH/Polysilasesquioxan SiVi in dem Beeich von 5/100 bis 100/100 liegt.

15. Verfahren nach einem der Ansprüche 8 bis 14, dadurch gekennzeichnet, daß das Zahlenverhältnis "SiH-Gruppen" zu "SiVi-Gruppen" in dem Bereich von 0,75/1 bis 4/1 liegt.

16. Verwendung der Mikrokugeln, die Gegenstand eines der Ansprüche 1 bis 7 sind oder erhalten werden nach dem Verfahren, welches Gegenstand eines der Ansprüche 8 bis 15 ist, als solche oder in wässriger Dispersion, als biologisch aktive Träger, mit der Maßgabe, daß die in Artikel 52 (4) EPÜ definierten therapeutischen Behandlungsverfahren ausgeschlossen sind.

## Patentansprüche (Patentansprüche für folgende(n) Vertragsstaat(en): ES)

1. Verfahren zur Herstellung von Mikroverbundkugeln, welches besteht aus:
- Dispergieren einer wässrigen Suspension von magnetisierbaren, nicht mit Dispergiermittel umhüllten Füllungen in einem organischen, nicht mit Wasser mischbaren Lösungmittel, wobei die Füllungen im allgemeinen eine Größe kleiner 300x10⁻⁴ »m, bevorzugt in dem Bereich von 50x10⁻⁴ bis 120x10⁻⁴ »m aufweisen,
- Lösen eines zu einem Polysilasesquioxan (SiVi) polykondensierbaren Alkoxysilans oder Alkoxysiloxans, welches pro Molekül mindestens zwei ethylenisch ungesättigte Gruppen ("SiVi-Gruppe") trägt, jede gebunden an ein Siliziumatom oder an ein über eine Si-C-Bindung an die Polysilasesquioxangruppe gebundenes Kohlenstoffatom einer organischen Gruppe, gegebenenfalls ionogene und/oder reaktive, nicht-ethylenische Gruppen trägt, ausgewählt aus den Amino-, Epoxy-, Mercapto- und Halogenresten, gebunden an ein Siliciumatom oder an ein Kohlenstoffatom einer über eine Si-C-Bindung an die Polysilasesquioxankette gebundenen Kohlenwasserstoffgruppe, in der organischen Phase der erhaltenen Suspension,
- Polykondensieren des genannten Alkoxysilans oder Alkoxysiloxans zu einen Polysilasesquioxan SiVi,
- Eliminieren des bei der Polykondensation erhaltenen Wassers,
- Lösen von mindestens einem Organopolysilan (Organohydrogenpolysiloxan) (genannt SiH), welches pro Molekül mindestens drei Wasserstoffatome, jedes gebunden an ein Siliziumatom, enthält, welches eine Viskosität in dem Bereich von 5 bis 1500 mPas bei 25°C aufweist und gegebenenfalls ionogene und/oder reaktive, nicht-vinylische Gruppen trägt, ausgewählt aus den Gruppen Amino, Epoxy, Mercapto und Halogen, gebunden an ein Siliziumatom oder ein über eine Si-C-Bindung an die Organopolysilankette (Organohydrogenpolysiloxankette) gebundenes Kohlenstoffatom einer Kohlenwasserstoffgruppe, sowie eines Hydrosilylierkatalysators,
- Vernetzen der Mischung aus den Polymeren SiVi und SiH,
- Abtrennen der magnetisierbaren Mikrokugeln,
- und gegebenenfalls erneutem Dispergieren der genannten Teilchen in Wasser.

2. Verfahren nach Anspruch 1, dadurch gekennzeichnet, daß die Konzentration der magnetisierbaren Füllungen in der wässrigen Suspension in dem Bereich von 0,5 bis 50 Gew. % ist.

3. Verfahren nach Anspruch 1 oder 2, dadurch gekennzeichnet, daß die Menge der verwendeten Füllungen so ist, daß das Gewichtsverhältnis von magnetisierbaren Füllungen/Alkoxysilan oder Alkoxysiloxan in dem Bereich von 0,005 bis 50 liegt.

4. Verfahren nach einem der Ansprüche 1 bis 3, dadurch gekennzeichnet, daß das Gewichtsverhältnis wässrige Phase/organische Phase in dem Bereich von 0,005 bis 2 ist.

5. Verfahren nach einem der Ansprüche 1 bis 4, dadurch gekennzeichnet, daß das Alkoxysilan die Formel (I):
R-Si-(OR′)₃ (I)
hat und das Alkoxysiloxan die Formel (II): 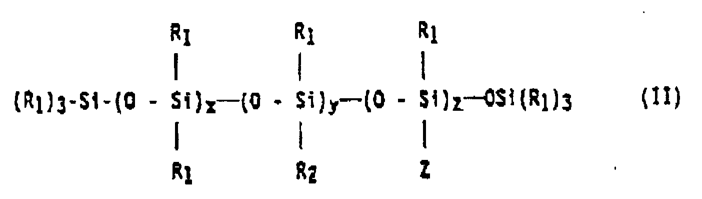 hat, wobei in den Formeln
- R bedeutet:
. einen Vinylrest
. einen ethylenisch ungesättigten Rest wie Methacryloxypropyl,
- R₁ bedeutet:
. einen C₁-C₃-Alkylrest
. einen Phenylrest
- R₂ bedeutet:
. einen R₁-Rest
. einen C₁-C₄-Alkylrest, substituiert mit reaktiven oder ionogenen Gruppen wie Amino-, Epoxy-, Mercapto-, Halogen
. einen Vinylrest, wobei die Zahl der Vinylreste mindestens zwei pro Molekül ist,
- OR′ ein OH-Rest ist oder ein hydrolysierbarer Rest wie solche, worin R′ bedeutet:
. einen C₁-C₄-Alkylrest
. einen -CO-CH₃-, -CO-C₂H₅-, -CO-CH₂-CO-CH₃, -CH₂-CH₂OH-, -CH₂CH₂-OCH₃-, -CH₂-CH₂-OC₂H₅-Rest
- Z einen -r-Si(R₁)-₃₋ₙ-(OR′)ₙ-Rest bedeutet, worin r eine C₁-C₁₈-Alkylengruppe ist, und n eine ganze Zahl von 0 bis 3 ist,
- die Symbole x, y, z einen ausreichenden Wert haben, um eine Viskosität niedriger als 100 mPas bei 25°C zu gewährleisten, wobei die Symbole x und y jeweils unabhängig voneinander Null sein können.

6. Verfahren nach einem der Ansprüche 1 bis 5, dadurch gekennzeichnet, daß das Organopolysilan (Organohydrogenpolysiloxan) SiH die Formel
Y (R₁)₂ SiO-(R₁ R˝ SiO)ₚ-(Y R₁ SiO)_{q}-Si (R₁)₂ Y III
hat, in welcher die Symbole R₁ gleich oder verschieden sind und wie in Anspruch 5 definiert sind, mit mindestens 80 % Methylresten, die Symbole y R¹ oder ein Wasserstoffatom darstellen, wobei die Zahl der Wasserstoffatome mindestens 3 pro Polymermolekül ist, die Symbole R˝ R₁ oder einen C₁-C₄-Alkylrest substituiert mit reaktiven oder ionogenen Gruppen, ausgewählt aus den Resten Amino, Epoxy, Mercapto, Halogen, darstellen, wobei die Symbole p und q solche sind, daß das genannte Polymer SiH eine Viskosität in dem Bereich von 5 bis 1500 mPas bei 25°C aufweist, und eine Zahl der ionogenen und/oder reaktiven, nicht-vinylischen Gruppen gegebenenfalls in dem Bereich von 1 bis 1000 pro Organopolysilanmolekül (Organohydrogenpolysiloxanmolekül) SiH und Polysilasesquioxanmolekül SiVi ist.

7. Verfahren nach einem der Ansprüche 1 bis 6, dadurch gekennzeichnet, daß das Gewichtsverhältnis von Organopolysilan (Organohydrogenpolysiloxan) SiH/Polysilasesquioxan SiVi in dem Beeich von 5/100 bis 100/100 liegt.

8. Verfahren nach einem der Ansprüche 1 bis 7, dadurch gekennzeichnet, daß das Zahlenverhältnis "SiH-Gruppen" zu "SiVi Gruppen" in dem Bereich von 0,75/1 bis 4/1 liegt.

9. Verwendung der Mikrokugeln als solche oder in wässriger Dispersion, erhalten nach dem Verfahren, welches Gegenstand eines der Ansprüche 1 bis 8 ist, als biologisch aktive Träger, unter der Bedingung, dass die Verfahren zur therapeutischen Behandlung gemaess Artikel 52 (4) des EPU ausgeschlossen sind.

## Claims (Claims for the following Contracting State(s): AT, BE, CH, DE, DK, FR, GB, GR, IT, LI, LU, NL, SE)

1. Magnetizable composite microspheres based on a crosslinked organosilicon polymer, occuring as such or in aqueous dispersion, characterized in that they are formed from :
- a core consisting of magnetizable fillers having a size generally smaller than 300 10⁻⁴ »m, preferably of the order of 50 to 120 10⁻⁴ »m, distributed uniformly inside a network of polysilsesquioxane (termed SiVi) containing, per molecule, at least two ethylenically unsaturated groups ("SiVi group") each bonded to a silicon atom or to a carbon atom of an organic group linked to the polysilsesquioxane chain by a Si-C bond, optionally carrying non-ethylenic ionogenic and/or reactive units chosen among the units amino, époxy, mercapto and halogeno bonded to a silicon atom or to a carbon atom of a hydrocarbon group linked to the polysilsesquioxane chain by a Si-C bond ; and
- a shell based on a crosslinked organosilicon polymer derived from the hydrosilylation
. of an organohydrogenopolysiloxane (termed SiH) containing, per molecule, at least three hydrogen atoms, each bonded to a silicon atom ("SiH groups"), having a viscosity of the order of 5 to 1,500 mPas at 25°C, and optionally carrying non-vinyl ionogenic and/or réactive units chosen among the units amino, époxy, mercapto and halogeno bonded to a silicon atom or to a carbon atom of a hydrocarbon group linked to the organohydrogenopoly-siloxane chain by a Si-C bond,
. with the ethylenically insaturated groups of said polysilsesquioxane SiVi.

2. Microspheres according to claim 1, characterized in that the amount of magnetizable fillers corresponds to about 0.25 to 95 % of the weight of the microspheres.

3. Microspheres according to claim 1 or 2, characterized in that the number-ratio of "SiH groups" to the "SiVi groups' is of the order of 0.75/1 to 4/1.

4. Microspheres according to any one of the preceding claims, characterized in that the ratio, by weight, of organohydrogenopolysiloxane SiH/polysilsesquioxane SiVi is of the order to 5/100 to 100/100.

5. Microspheres according to any one of the preceding claims, characterized in that the polysilsesquioxane SiVi is derived from the polycondensation of an alkoxysilane of formula :
R Si (OR′)₃ (I)
or 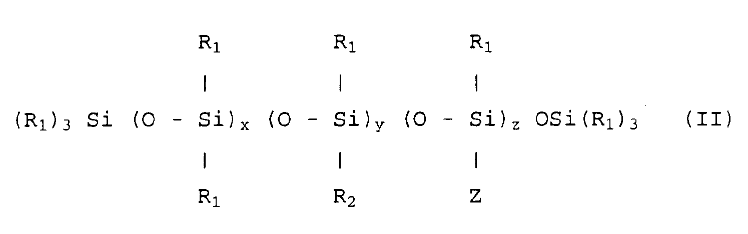 in which formulae
- R represents
. a vinyl radical, or
. an ethylenically unsaturated radical such as methacryloxypropyl, ...
- R₁ represents
. a C₁-C₃ alkyl radical, or
. a phenyl radical,
- R₂ represents
. a radical R₁,
. a C₁-C₄ alkyl radical substituted by reactive or ionogenic groups, such as amino, epoxy, mercapto, halogeno, ... or
. a vinyl radical, the number of vinyl radicals being at least two per molecule,
- OR′ is an OH radical or a hydrolysable radical such as those in which R′ represents
. a C₁-C₄ alkyl radical or
. a -CO-CH₃, -CO-C₂H₅, -CO-CH₂-CO-CH₃, -CH₂ CH₂OH, - CH₂CH₂ OCH₃, -CH₂-CH₂ OC₂H₅ ... radical,
- Z represents a radical -r-Si(R₁)₃₋ₙ (OR′)ₙ, in which r is a C₁-C₁₈ alkylene group, and
- the symbols x, y and z have a value sufficient to ensure a viscosity lower than 100 mPas at 25°C it being possible for the symbols x and y separately to be zero.

6. Microspheres according to any one of the preceding claims, characterized in that the organohydrogenopolysiloxane SiH has the formula
Y (R₁)₂ SiO (R₁ R˝ SiO)ₚ (Y R₁ SiO)_{q} Si (R₁)₂ Y (III)
in which formula the symbols R₁ are identical or different and have the definition given above, with at least 80 % of methyl radicals, the symbols Y represent R₁ or a hydrogen atom, the number of hydrogen atoms being at least 3 per molecule of polymer, the symbols R˝ represent R₁ or a C₁-C₄ alkyl radical substituted by reactive or ionogenic group chosen among the units amino, epoxy, mercapto, halogeno ..., the symbols p and q being such that the said polymer SiH has a viscosity of the order of 5 to 1,500 mPas at 25°C, and a number of optional non-vinyl ionogenic and/or reactive units ranging from 1 to 1,000 per molecule of organohydrogenopolysiloxane SiH and of polysilsesquioxane SiVi.

7. Microspheres according to any one of the preceding claims, characterized in that they have a particle size distribution of the order of 0.05 to 3 microns.

8. Process for the preparation of composite microspheres, consisting in :
- dispersing an aqueous suspension of magnetizable fillers not coated with dispersing agent, said fillers having a size generally smaller than 300 10⁻⁴ »m, preferably of the order of 50 10⁻⁴ to 120 10⁻⁴ »m, in a water-immiscible organic solvent,
- dissolving an alkoxysiloxane, capable of undergoing polycondensation to form a polysilsesquioxane (SiVi) containing, per molecule, at least two ethylenically unsaturated groups ("SiVi group"), each bonded to a silicon atom or to a carbon atom of an organic group linked to the polysilsesquioxane chain by a Si-C bond, optionally carrying non-ethylenic ionogenic and/or reactive units chosen among the units amino, époxy, mercapto and halogeno bonded to a silicon atom or to a carbon atom of a hydrocarbon group linked to the polysilsesquioxane chain by an Si-C bond, in the organic phase of the dispersion obtained,
- subjecting the said alkoxysiloxane to polycondensation to a polysilsesquioxane SiVi,
- removing the water resulting from the polycondensation,
- dissolving at least one organohydrogenopolysiloxane (termed SiH) containing, per molecule, at least three hydrogen atom each linked to a silicon atom, having a viscosity of the order of 5 to 1,500 mPas at 25°C, and optionally carrying non-vinyl ionogenic and/or reactive units chosen among the units amino, époxy, mercapto and halogeno bonded to a silicon atom or to a carbon atom of a hydrocarbon group linked to the organohydrogenopolysiloxane chain by a Si-C bond, and also a hydrosilylation catalyst in the organic phase of the dispersion obtained,
- crosslinking the mixture of polymers SiVi and SiH,
- separating off the magnetizable microspheres, and
- if appropriate, redispersing the said particles in water.

9. Process according to claim 8, characterized in that the concentration of magnetizable fillers in the aqueous suspension is of the order of 0.5 to 50 % by weight.

10. Process according to claim 8 or 9, characterized in that the amount of fillers used is such that the ratio, by weight, of magnetizable fillers/alkoxysilane is of the order of 0.005 to 50.

11. Process according to any one of claims 8 to 10, characterized in that the ratio, by weight, of aqueous phase/organic phase is of the order of 0.005 to 2.

12. Process according to any one of claims 8 to 11, characterized in that the alkoxysilane has the formula
R Si (OR′)₃ (I)
or 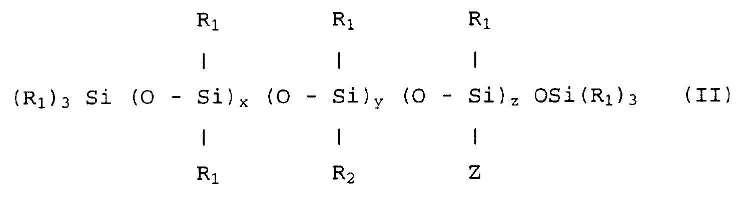 in which formulae
- R represents
. a vinyl radical, or
. an ethylenically unsaturated radical such as methacryloxypropyl, ...
- R₁ represents
. a C₁-C₃ alkyl radical, or
. a phenyl radical,
- R₂ represents
. a radical R₁,
. a C₁-C₄ alkyl radical substituted by reactive or ionogenic groups, such as amino, epoxy, mercapto, halogeno, ... or
. a vinyl radical, the number of vinyl radicals being at least two per molecule,
- OR′ is an OH radical or a hydrolysable radical such as those in which R′ represents
. a C₁-C₄ alkyl radical or
. a -CO-CH₃, -CO-C₂H₅, -CO-CH₂-CO-CH₃, -CH₂ CH₂OH, - CH₂CH₂ OCH₃, -CH₂-CH₂ OC₂H₅ ... radical,
- Z represents a radical -r-Si(R₁)₃₋ₙ (OR′)ₙ, in which r is a C₁-C₁₈ alkylene group, and
- the symbols x, y and z have a value sufficient to ensure a viscosity lower than 100 mPas at 25°C it being possible for the symbols x and y separately to be zero.

13. Process according to any one of claims 8 to 12, characterized in that the organohydrogenopolysiloxane SiH has the formula :
Y (R₁)₂ SiO (R₁ R˝ SiO)ₚ (Y R₁ SiO)_{q} Si (R₁)₂ Y (III)
in which formula the symbols R₁ are identical or different and have the definition given above, with at least 80 % of methyl radicals, the symbols Y represent R₁ or a hydrogen atom, the number of hydrogen atoms being at least 3 per molecule of polymer, the symbols R˝ represent R₁ or a C₁-C₄ alkyl radical substituted by reactive or ionogenic group chosen among the units amino, epoxy, mercapto, halogeno ..., the symbols p and q being such that the said polymer SiH has a viscosity of the order of 5 to 1,500 mPas at 25°C, and a number of optional non-vinyl ionogenic and/or reactive units ranging from 1 to 1,000 per molecule of organohydrogenopolysiloxane SiH and of polysilsesquioxane SiVi.

14. Process according to any one of claims 8 to 13, characterized in that the ratio, by weight, of organohydrogenopolysiloxane SiH/polysilsesquioxane SiVi is of the order of 5/100 to 100/100.

15. Process according to any one of claims 8 to 14, characterized in that the number-ratio of "SiH groups" to the "SiVI" groups" is of the order of 0.75/1 to 4/1.

16. Use of the microspheres, as such or in aqueous dispersion, which are the subject of any one of claims 1 to 7 or obtained according to the process which is the subject of any one of claims 8 to 15 as active supports in biology proviso that the methods of therapeutic treatment such as defined at article 52(4) EPC be disclaimed.

## Claims (Claims for the following Contracting State(s): ES)

1. Process for the preparation of composite microspheres, consisting in :
- dispersing an aqueous suspension of magnetizable fillers not coated with dispersing agent, said fillers having a size generally smaller than 300 10⁻⁴ »m, preferably of the order of 50 10⁻⁴ to 120 10⁻⁴ »m, in a water-immiscible organic solvent,
- dissolving an alkoxysiloxane, capable of undergoing polycondensation to form a polysilsesquioxane (SiVi) containing, per molecule, at least two ethylenically unsaturated groups ("SiVi group"). each bonded to a silicon atom or to a carbon atom of an organic group linked to the polysilsesquioxane chain by a Si-C bond, optionally carrying non ethylenic ionogenic and/or reactive units chosen among the units amino, époxy, mercapto and halogeno bonded to a silicon atom or to a carbon atom of a hydrocarbon group linked to the polysilsesquioxane chain by an Si-C bond, in the organic phase of the dispersion obtained,
- subjecting the said alkoxysiloxane to polycondensation to a polysilsesquioxane SiVi,
- removing the water resulting from the polycondensation,
- dissolving at least one organohydrogenopolysiloxane (termed SiH) containing, per molecule, at least three hydrogen atom each linked to a silicon atom, having a viscosity of the order of 5 to 1,500 mPas at 25°C, and optionally carryng non-vinyl ionogenic and/or reactive units chosen among the units amino, époxy, mercapto and halogeno bonded to a silicon atom or to a carbon atom of a hydrocarbon group linked the organohydrogenopolysiloxane chain by a Si-C bond, and also a hydrosilylation catalyst in the organic phase of the dispersion obtained,
- crosslinking the mixture of polymers SiVi and SiH,
- separating off the magnetizable microspheres, and
- if appropriate, redispersing the said particles in water.

2. Process according to claim 1, characterized in that the concentration of magnetizable fillers in the aqueous suspension is of the order of 0.5 to 50 % by weight.

3. Process according to claim 1 or 2, characterized in that the amount of fillers used is such that the ratio, by weight, of magnetizable fillers/alkoxysilane is of the order of 0.005 to 50.

4. Process according to any one of claims 1 to 3, characterized in that the ratio, by weight, of aqueous phase/organic phase is of the order of 0.005 to 2.

5. Process according to any one of claims 1 to 4, characterized in that the alkoxysilane has the formula
R Si (OR′)₃ (I)
or 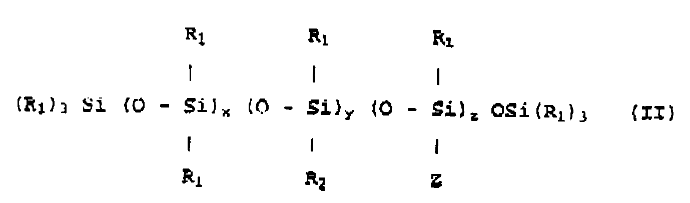 in which formulae
- R represents
. a vinyl radical, or
. an ethylenically unsaturated radical such as methacryloxypropyl, ...
- R₁ represents
. a C₁-C₃ alkyl radical, or
. a phenyl radical,
- R₂ represents
. a radical R₁,
. a C₁-C₄ alkyl radical substituted by reactive or ionogenic groups, such as amino, epoxy, mercapto, halogeno, ... or
. a vinyl radical, the number of vinyl radicals being at least two per molecule,
- OR′ is an OH radical or a hydrolysable radical such as those in which R′ represents
. a C₁-C₄ alkyl radical or
. a -CO-CH₃, -CO-C₂H₅, -CO-CH₂-CO-CH₃, -CH₂ CH₂OH, - CH₂CH₂ OCH₃, -CH₂-CH₂ OC₂H₅ ... radical,
- Z represents a radical -r-Si(R₁)₃₋ₙ (OR′)ₙ, in which r is a C₁-C₁₈ alkylene group, and
- the symbols x, y and z have a value sufficient to ensure a viscosity lower than 100 mPas at 25°C it being possible for the symbols x and y separately to be zero.

6. Process according to any one of claims 1 to 5, characterized in that the organohydrogenopolysiloxane SiH has the formula :
Y (R₁)₂ SiO (R₁ R˝ SiO)ₚ (Y R₁ SiO)_{q} Si (R₁)₂ Y (III)
in which formula the symbols R₁ are identical or different and have the definition given above, with at least 80 % of methyl radicals, the symbols Y represent R₁ or a hydrogen atom, the number of hydrogen atoms being at least 3 per molecule of polymer, the symbols R˝ represent R₁ or a C₁-C₄ alkyl radical substituted by reactive or ionogenic group chosen among the units amino, epoxy, mercapto, halogeno ..., the symbols p and q being such that the said polymer SiH has a viscosity of the order of 5 to 1.500 mPas at 25°C, and a number of optional non-vinyl ionogenic and/or reactive units ranging from 1 to 1.000 per molecule of organohydrogenopolysiloxane SiH and of polysilsesquioxane SiVi.

7. Process according to any one of claims 1 to 6, characterised in that the ratio, by weight, of organo hydrogenopolysiloxane SiH/polysilsesquioxane SiVi is of the order of 5/100 to 100/100.

8. Process according to any one of claims 1 to 7, characterized in that the number-ratio of "SiH groups" to the "SiVI" groups" is of the order ot 0.75/1 to 4/1.

9. Use of the microspheres, as such or in aqueous dispersion, obtained according to the process which is the subject of any one of claims 1 to 8 as active supports in biology, with the proviso that methods for therapeutical treatment according to Article 52 (4) of the EPC be excluded.
